# EUROPEAN PATENT APPLICATION

(11) **EP 4 430 952 A1**
(43) Date of publication of application: **18.09.2024**
(21) Application number: 22901756.1
(22) Date of filing: 30.11.2022
(51) Int. Cl.: A23F 3/30, A23F 3/32, A23F 3/40, A23L 2/56, A23L 2/60

(54) **MILK TEA PREPARATION COMPOSITION AND PREPARATION METHOD THEREFOR**

(30) Priority: 30.11.2021 KR 20210168975
(71) Applicant: CJ CheilJedang Corporation, Seoul 04560 (KR)
(72) Inventor: KIM, Sunny, Seoul 04560 (KR); LEE, Minhyun, Seoul 04560 (KR); JUNG, Jiwoo, Seoul 04560 (KR); KONG, Moon Hee, Seoul 04560 (KR); KIM, Chul Jin, Seoul 04560 (KR)
(74) Representative: Mewburn Ellis LLP
(86) International application number: PCT/KR2022/019174
(87) International publication number: WO 2023/101398

(57) **Abstract**

The present disclosure relates to a composition for preparing milk tea, milk tea comprising the composition, and a method for preparing the composition for preparing milk tea.

## Description

### [Technical Field]

The present disclosure relates to a composition for preparing milk tea, milk tea comprising the composition, and a method for preparing the composition for preparing milk tea.

### [Background Art]

Milk tea refers to various types of beverages in which black tea is added to milk or tea and milk are mixed. An instant milk tea composition is provided by mixing instant black tea powder instead of brewing black tea with sugar.

Milk tea is intended to enjoy the flavor and taste peculiar to black tea and is prepared by mixing black tea powder, sugar and the like and dissolving the mixture in warm or cold milk. However, black tea extract powder is an insoluble ingredient and has a problem in that the powder remains in the beverage because of its low solubility during beverage preparation, and this not only impairs the taste and flavor but also causes a dull and unpleasant mouthfeel due to powder particles that are not evenly dispersed. Additionally, in the case of preparing a beverage using instant black tea extract powder, the increase in tannin content due to extraction causes bitter and astringent tastes, and this has a negative impact on consumer preference.

In order to solve the above-mentioned problems, a method in which sweeteners such as sugar, additives, or flavorings are added to instant milk tea compositions is generally used, but a problem arises in that the added sweeteners increase the content of saccharides in beverages and calories, the large amount of sugar increases the sticky and heavy mouthfeel resulting in reduced preference and decreases the solubility, and the unique flavor of black tea is deteriorated by excessive use of additives.

### [citation List]

### [Patent Document]

Patent Document 0001: Korean Patent Publication No. 10-2148188

### [Disclosure]

### [Technical Problem]

The present inventors have developed a composition for preparing milk tea that has reduced bitter and astringent tastes peculiar to black tea extract powder and improved solubility and dispersibility and mouthfeel, milk tea comprising the composition, and a method for preparing the composition for preparing milk tea, and thus completed the present disclosure.

### [Technical Solution]

An object of the present disclosure is to provide a composition for preparing milk tea comprising black tea extract powder, xylose sugar, sugar alcohol, and a natural high-intensity sweetener.

Another object of the present disclosure is to provide milk tea comprising the composition.

Still another object of the present disclosure is to provide a method for preparing a composition for preparing milk tea.

Still another object of the present disclosure is to provide a composition for preparing milk tea prepared by the method.

Still another object of the present disclosure is to provide milk tea comprising the composition.

### [Advantageous Effects]

The composition for preparing milk tea of the present disclosure has an excellent effect of preparing milk tea that has reduced bitter and astringent tastes peculiar to black tea extract powder and improved solubility and dispersibility and mouthfeel, and thus increases consumer preference by replacing sugar with sugar alcohol and a natural high-intensity sweetener at a certain ratio.

### [Brief Description of Drawings]

FIG. 1 is a diagram illustrating the solubility measurement results of powders for preparing milk tea; and
FIG. 2 is a diagram illustrating the sensory evaluation results of powders for preparing milk tea.

### [Detailed Description of Preferred Embodiments]

The present disclosure will be described in detail as follows. Meanwhile, each description and embodiment disclosed in this disclosure may also be applied to other descriptions and embodiments. That is, all combinations of various elements disclosed in this disclosure fall within the scope of the present disclosure. Further, the scope of the present disclosure is not limited by the specific description described below. Further, a number of papers and patent documents are referenced and cited throughout this specification. The disclosures of the cited papers and patent documents are incorporated herein by reference in their entirety to further clarify the level and scope of the subject matter to which the present disclosure pertains.

An aspect of the present disclosure provides a composition for preparing milk tea comprising black tea extract powder, xylose sugar, sugar alcohol, and a natural high-intensity sweetener.

Specifically, the xylose sugar and the natural high-intensity sweetener may be comprised in the form of a core-shell structure in which the xylose sugar is coated with the natural high-intensity sweetener, but are not limited thereto.

As an embodiment of the present disclosure, by spray-coating trace amounts of non-digestible (or indigestible) maltodextrin, enzyme-treated stevia, and stevioside into a core-shell, the sweet taste can be evenly distributed over the entire product and the quality of the product can be maintained. Additionally, it is possible to have a particle size and surface area that is advantageous for the improvement of solubility.

The composition for preparing milk tea of the present disclosure has an excellent effect of preparing milk tea that has reduced bitter and astringent tastes peculiar to black tea extract powder and improved solubility and dispersibility and mouthfeel, and thus increases consumer preference by replacing sugar with sugar alcohol and a natural high-intensity sweetener at a certain ratio and comprising a core-shell structure in which the xylose sugar is coated with the natural high-intensity sweetener.

Specifically, the black tea extract powder may be comprised at 1 to 10 parts by weight, specifically at 5 to 10 parts by weight, more specifically at 9 to 10 parts by weight based on 100 parts by weight of the total weight of the composition, but is not limited thereto.

Black tea powder is a product prepared by extracting black tea and performing drying and pulverizing the obtained extract, and is an insoluble dark red powder that contains tannin and has bitter and astringent tastes. Black tea powder is widely used in beverages and bakery products.

Specifically, the xylose sugar may be comprised at 30 to 90 parts by weight, specifically at 35 to 70 parts by weight, more specifically at 40 to 65 parts by weight based on 100 parts by weight of the total weight of the composition, but is not limited thereto.

The term "xylose sugar" of the present invention refers to xylose-containing sugar that maintains sweetness characteristics at the same level compared to sugar, starch syrup, fructose, and the like, inhibits the decomposition of sugar, and thus slows down the absorption of sugar in the body. Specifically, the xylose sugar may be a mixture of sucrose and xylose, for example, one prepared by mixing sucrose and xylose at a weight ratio of 1 : 0.05 to 1 : 0.5, but is not limited thereto.

Xylose is a natural sweetener found in birch trees, corn, and the like, has a degree of sweetness of about 40% of that of sugar, and is known as one of several sweeteners that can supplement sugar and compensate for the harmful effects of sugar. In the case of being consumed simultaneously with sugar, xylose interferes with the decomposition of sugar by inhibiting the activity of sucrase, a sugar-decomposing enzyme, thus has the effect of inhibiting the absorption of sugar into the body and excreting sugar out of the body, and is recognized as being effective in preventing sudden increases in blood sugar levels and adult diseases such as diabetes and obesity. In addition, arabinoxylan, a complex polysaccharide composed of arabinose and xylose, is a bioactive substance related to anti-allergy, immune activity, and anti-cancer, and results of various studies on arabinoxylan have been published recently.

Specifically, the sugar alcohol may be comprised at 0.1 to 50 parts by weight, specifically at 0.5 to 40 parts by weight, more specifically at 1 to 30 parts by weight based on 100 parts by weight of the total weight of the composition, but is not limited thereto.

By comprising the sugar alcohol, the content of saccharides can be decreased.

In addition, the sugar alcohol may be one or more selected from the group consisting of erythritol, tagatose, xylose, arabinose, ribose, xylitol, lactitol, maltitol, and sorbitol, specifically erythritol, but is not limited thereto.

The erythritol is a sugar alcohol known to have a sweet taste profile most similar to white sugar, and is a substance having very minimal calories of 0 to 0.2 kcal/g. Accordingly, in the case of comprising the erythritol, the composition can be usefully used in the preparation of low-calorie products.

Specifically, the natural high-intensity sweetener may be comprised at 0.01 to 0.1 parts by weight based on 100 parts by weight of the total weight of the composition, but is not limited thereto.

The natural high-intensity sweetener is comprised at 0.01 to 0.1 parts by weight, specifically at 0.020 to 0.095 parts by weight, more specifically at 0.025 to 0.090 parts by weight based on 100 parts by weight of the total weight of the composition, so that the composition has a degree of sweetness similar to that when the same amount of sugar is used. When the amount of natural high-intensity sweetener added is too small, the degree of sweetness is lower than that of sugar, and the effect of increasing the degree of sweetness achieved by mixing a high-intensity sweetener cannot be exerted. However, when the amount of natural high-intensity sweetener added is too large, a bitter taste peculiar to high-intensity sweeteners is produced since these sweetening agents do not provide the exactly same sweet taste as that of sugar although these sweetening agents are products having improved bitter taste and aftertaste. The content of high-intensity sweeteners is thus limited to the above-mentioned values considering that it is not appropriate to use the high-intensity sweeteners in too small or too large in an amount.

In the case where the natural high-intensity sweetener is comprised at the above content, it is possible to mask off-taste and off-flavor of the composition, and the solubility and dispersibility are improved during spray coating into a core-shell form.

In addition, the natural high-intensity sweetener may be one or more selected from the group consisting of enzyme-treated stevia, stevioside, sucralose, aspartame, a monk fruit extract, a licorice extract, and thaumatin, specifically, enzyme-treated stevia and/or stevioside, but is not limited thereto.

The enzyme-treated stevia (Glucosyl-Stevia) is obtained by adding glucose to stevioside using glycosyltransferase, and is a taste-improved product obtained by removing the bitter taste and aftertaste unique to stevioside.

Stevioside is a high-intensity sweetener classified as a natural additive, and is a natural sweetener produced by extracting the leaves of stevia (plant of the family *Asteraceae, Stevia rebaudiana Bertoni)* and refining the extract, and is a heat-and pH-stable sweetener, and its sweetness is about 200 times that of sugar. However, even though stevioside has a superior sweetness compared to sugar, it has disadvantages of leaving a long-lasting aftertaste and having a bitter and unpleasant taste in addition to the sweet taste. The stevioside may be stevioside, rebaudioside A, rebaudioside B, rebaudioside C, rebaudioside D, rebaudioside E, rubusoside, dulcoside A, stevioside, or a mixture thereof, but is not limited thereto, and any high-intensity sweetener may be used, as long as it is used by those skilled in the art to impart sweetness.

The natural high-intensity sweetener is known to have its own bitter taste. Therefore, the bitter taste of the high-intensity sweetener may be reduced and its preference may be increased by mixing with the sugar alcohol, specifically, erythritol.

In the case where the sugar, sugar alcohol, and natural high-intensity sweetener are mixed with black tea extract powder at the above contents, the tannin component that produces the bitter taste of black tea can be effectively masked. Accordingly, it is possible to provide a natural sugar-free composition that can improve the taste quality, mouthfeel, and solubility and dispersibility of conventional composition products while significantly reducing calories.

The composition for preparing milk tea of the present disclosure may further comprise polysaccharides.

Specifically, the polysaccharides may be polysaccharides such as dextrins, maltodextrin, cyclodextrins, fructooligosaccharides, isomaltooligosaccharides, xylooligosaccharides, gentio-oligosaccharides, maltooligosaccharides, or galactooligosaccharides, more specifically maltodextrin, but is not limited thereto.

The maltodextrin is a type of polysaccharide; is used as an excipient; may be used by adjusting its content depending on the form of use of the composition for preparing milk tea, for example, powder form; and may be comprised at 5 to 30 parts by weight based on 100 parts by weight of the total weight of the composition although the content thereof is not limited thereto.

The composition for preparing milk tea of the present disclosure may further comprise a flavoring agent, specifically, a flavoring agent for black tea flavor. The flavoring agent for black tea flavor may be comprised at 0.1 to 1.5 parts by weight based on 100 parts by weight of the total weight of the composition although the content thereof is not limited thereto.

When the flavoring agent is added to the composition for preparing milk tea of the present disclosure, the black tea flavor of milk tea can be enriched, and a unique flavor can be offered when milk tea is prepared by adding the flavoring agent at 0.1 to 1.5 parts by weight. The flavor is weakened when the composition for preparing milk tea is mixed with water or milk because of its nature, so the unique flavor of black tea is not felt when the intensity of flavor is weak. Therefore, the ratio of flavoring agent added is selected within the above range to offer an appropriate intensity of flavor without causing displeasure. In the case where the flavoring agent is added in excess of the above range, the unique chemical off-flavor is exerted and the product quality may deteriorate. As the flavoring agent, a commercially available flavoring agent that can be generally used by those skilled in the art may be purchased and used, or a flavoring agent may be prepared and used depending on the purpose.

Specifically, the composition for preparing milk tea of the present disclosure may further comprise functional ingredients, specifically saponins, antioxidants, dietary fiber sources, fatty acids, vitamins, glucosamine, minerals, preservatives, hydrating agents, probiotics, prebiotics, agents for weight management, agents for osteoporosis management, phytoestrogens, long-chain primary aliphatic saturated alcohols, phytosterols, and combinations thereof, more specifically vitamin C.

In the case where vitamin C is comprised in the composition for preparing milk tea of the present disclosure, vitamin C makes the composition have specific physiological functions such as radical removal and anti-aging. The content of vitamin C is not limited to this, but vitamin C may be comprised at 0.1 to 1.5 parts by weight based on 100 parts by weight of the total weight of the composition.

In the case where vitamin C is comprised in the above range, vitamin C can function as an antioxidant without reducing organoleptic properties.

The composition for preparing milk tea of the present disclosure may further comprise an acidity regulator, a stabilizer, sodium caseinate, refined salt, and the like in addition to the black tea extract powder, xylose sugar, sugar alcohol, natural high-intensity sweeteners, polysaccharides, flavoring agents, and functional ingredients. In addition to these, the composition for preparing milk tea of the present disclosure may further comprise known materials that are generally used to prepare compositions for preparing milk tea.

The form of the composition for preparing milk tea of the present disclosure is not limited as long as it is a composition suitable for preparing milk tea, and may be a powder, liquid, or solid, specifically a powder.

Still another aspect of the present disclosure provides milk tea comprising the composition described above.

The composition is as described above.

Specifically, the milk tea may further comprise water, soy milk or milk, specifically milk in addition to the composition for preparing milk tea.

Specifically, the milk tea may have reduced bitter and astringent tastes compared to milk tea that does not comprise xylose sugar, sugar alcohol, and a natural high-intensity sweetener.

Astringent taste is an unpleasant taste caused by temporary denaturation and coagulation of viscous proteins on the surface of the tongue and paralysis of the gustatory nerves, and milk tea has a unique astringent and sour aftertaste because of the tannin component in black tea leaves. The milk tea of the present disclosure is advantageous in that the astringent taste felt in the mouth is reduced without separately removing the tannin component by the addition of xylose sugar, sugar alcohol, and a natural high-intensity sweetener.

The milk tea may have improved solubility and dispersibility and mouthfeel compared to milk tea that does not comprise the core-shell structure and the reduced bitter taste of black tea extract powder.

Still another aspect of the present disclosure provides a method for preparing the composition for preparing milk tea.

The composition for preparing milk tea is as described above.

Specifically, a method for preparing a composition for preparing milk tea is provided which comprises preparing a diluted solution by dissolving maltodextrin and a natural high-intensity sweetener in water; preparing a core-shell structure in which xylose sugar is present as a core and the core is coated with the natural high-intensity sweetener by spraying and coating the xylose sugar with the diluted solution; drying the core-shell with hot air in a temperature range of 70°C to 80°C for 10 to 30 minutes; cooling the core-shell through treatment with cooling air at 1°C to 50°C for 10 to 30 minutes; and preparing a composition by stirring the cooled core-shell, sugar alcohol, and black tea extract powder.

Specifically, the black tea extract powder may be comprised at 1 to 10 parts by weight based on 100 parts by weight of the total weight of the composition, the xylose sugar may be comprised at 30 to 90 parts by weight based on 100 parts by weight of the total weight of the composition, the sugar alcohol may be comprised at 0.1 to 50 parts by weight based on 100 parts by weight of the total weight of the composition, and the natural high-intensity sweetener may be comprised at 0.01 to 0.1 parts by weight based on 100 parts by weight of the total weight of the composition, but the contents are not limited thereto.

Hereinafter, the method for preparing a composition for preparing milk tea of the present disclosure will be described in detail.

First, a diluted solution is prepared by dissolving maltodextrin and a natural high-intensity sweetener in water.

Next, a core-shell structure in which xylose sugar is present as a core and the core is coated with the natural high-intensity sweetener is prepared by spraying and coating the xylose sugar with the diluted solution. The xylose sugar may be prepared by mixing sugar and xylose at a weight ratio of 1 : 0.05 to 1 : 0.5, but is not limited thereto.

The composition for preparing milk tea of the present disclosure has an excellent effect of preparing milk tea that has reduced bitter and astringent tastes peculiar to black tea extract powder and improved solubility and dispersibility and mouthfeel, and thus increases consumer preference by comprising a core-shell structure in which the xylose sugar is coated with the natural high-intensity sweetener.

As an embodiment of the present disclosure, by spray-coating trace amounts of non-digestible maltodextrin, enzyme-treated stevia, and stevioside into a core-shell, the sweet taste can be evenly distributed over the entire product and the quality of the product can be maintained. Additionally, it is possible to have a particle size and surface area that is advantageous to the improvement of solubility. Specifically, by first mixing sugar and xylose and then spray-coating the mixture with a diluted solution of non-digestible maltodextrin and a natural high-intensity sweetener, the surface of the particles is formed so that the dissolution rate can rapidly increase.

Next, the core-shell is dried with hot air in a temperature range of 70°C to 80°C, specifically 75°C for 10 to 30 minutes, specifically 20 minutes.

In the case where the composition for preparing milk tea of the present disclosure is in a powder form, as the moisture content increases, microbial growth is activated and this leads to quality deterioration. Therefore, through the drying step, a moisture condition that can inhibit microbial growth in the product is created, and mixing with other raw materials can be performed under an appropriate condition in the next process step. The sugar melts and a core-shell cannot be formed when drying is performed at a temperature higher than the above temperature range, and a moisture condition suitable for microbial growth is created and this may lead to quality deterioration when drying is performed at a temperature lower than the above temperature range.

Next, the core-shell is cooled through treatment with cooling air at 1°C to 50°C, specifically 40°C or less, for 10 to 30 minutes, specifically 10 minutes.

When the core-shell is not cooled after the hot air drying step, the core-shell melts together with other raw materials and a suitable product cannot be formed. In the preparation method of the present disclosure, the temperature and time are set to be optimal for energy efficiency during the process, cooling does not proceed under temperature and time conditions exceeding the above conditions, and the size of core-shell crystals increases and the improvement in solubility is reduced in a temperature range lower than the above condition.

Next, a composition is prepared by stirring the cooled core-shell, sugar alcohol, and black tea extract powder.

Specifically, the step of preparing a composition by stirring the cooled core-shell, sugar alcohol, and black tea extract powder may further comprise adding maltodextrin, a flavoring agent, or vitamin C, but is not limited thereto.

For example, a composition may be prepared by stirring the cooled core-shell, sugar alcohol, maltodextrin, black tea extract powder, and a flavoring agent.

Still another aspect of the present disclosure provides a composition for preparing milk tea that is prepared by the method described above.

The method and composition for preparing milk tea are as described above.

Still another aspect of the present disclosure provides milk tea comprising the composition described above.

The method and composition for preparing milk tea are as described above.

Specifically, the milk tea may have reduced bitter and astringent tastes compared to milk tea that does not comprise xylose sugar, sugar alcohol, and a natural high-intensity sweetener, but is not limited thereto.

Specifically, the milk tea may have improved solubility and dispersibility and mouthfeel compared to milk tea comprising a composition prepared by a method that does not comprise a spraying and coating process, but is not limited thereto.

### [Mode for Carrying Out the Invention]

Hereinafter, the present disclosure will be described in more detail by way of exemplary embodiments. However, the following exemplary embodiments are only preferred embodiments for illustrating the present disclosure, and thus are not intended to limit the scope of the present disclosure thereto. Meanwhile, technical matters not described in the present specification may be sufficiently understood and easily implemented by those skilled in the technical field of the present disclosure or similar technical fields.

### Example: Preparation of powder for preparing milk tea

Powders for preparing milk tea of Comparative Example and Examples 1 to 5 were prepared according to the compositions shown in Table 1 below.

Specifically, in the case of Examples 1 to 5, white sucrose (CJ CheilJedang) and xylose (DUPONT) were mixed at a 10 : 1 ratio in a zero gravity mixer for 5 minutes to prepare xylose sugar.

Next, non-digestible maltodextrin (Samyang Genex), enzyme-treated stevia (Daepyeong), and steviol glycosides (Daepyeong, purity 98% or more) were quantitatively measured and diluted in purified water to prepare a diluted solution, and the mixture of white sucrose and xylose was sprayed and coated with the diluted solution.

Next, drying was performed by blowing hot air (75°C or more) for 20 minutes or more, and then cooling was performed by blowing cooling air at 40°C or less for 10 minutes.

Accordingly, the core must comprise a mixture of white sucrose and xylose, and the coating liquid (shell) must comprise non-digestible maltodextrin, enzyme-treated stevia, and stevioside.

Next, sugar alcohol, maltodextrin (QINHUANG DAO LIHUA STARCH CO.LTD), black tea extract powder (NESTLE INDIA), a flavoring agent (HANBIT FLAVOR&FRAGRANCE CO., LTD.), and vitamin C (HEBEI WELCOME PHARAMACEUTICAL CO.LTD) were quantitatively measured and prepared, and then the prepared raw materials were sequentially added into a zero gravity mixer (F20, SEJI TECH CO., LTD.) and continuously stirred at 40 rpm.

Next, after all the raw materials were added, stirring was continuously performed for 10 minutes at the same speed so that the raw materials were uniformly dispersed and mixed, and the mixture was recovered and used as Examples 1 to 5.

Meanwhile, in the case of Comparative Example, white sucrose (CJ CheilJedang), maltodextrin (QINHUANG DAO LIHUA STARCH CO.LTD), black tea extract powder (NESTLE INDIA), a flavoring agent (HANBIT FLAVOR&FRAGRANCE CO., LTD.), and vitamin C (HEBEI WELCOME PHARAMACEUTICAL CO.LTD) were quantitatively measured and prepared according to the contents in Table 1 below, then added into a zero gravity mixer (F20, SEJI TECH CO., LTD.), and stirred at 40 rpm. After all the raw materials were added, stirring was performed for 10 minutes at the same speed.

Meanwhile, the fine grain sugar (CJ CheilJedang, fine grain sugar, purity 98% or higher) in Comparative Example means sucrose obtained by pulverizing general sucrose to have a small particle size.

**[Table 1]**

| **Name of raw material** | **Comparative Example** | **Example 1** | **Example 2** | **Example 3** | **Example 4** | **Example 5** |
|---|---|---|---|---|---|---|
| **Fine grain sugar** | 73.50 | - | - | - | - | - |
| **Xylose sugar** | - | 73.000 | 61.000 | 56.500 | 52.000 | 45.000 |
| **Maltodextrin** | 15.58 | - | 22.995 | 22.490 | 16.995 | 13.990 |
| **Erythritol (sugar alcohol)** | - | 1.000 | 5.000 | 10.000 | 20.000 | 30.000 |
| **Enzyme-treated stevia (natural high-intensity sweetener 1)** | - | 0.015 | 0.070 | 0.070 | 0.060 | 0.065 |
| **Stevioside (natural high-intensity sweetener 2)** | - | 0.010 | 0.015 | 0.020 | 0.025 | 0.025 |
| **Black tea extract powder** | 9.70 | 9.700 | 9.700 | 9.700 | 9.700 | 9.700 |
| **Black tea flavor** | 1.20 | 1.200 | 1.200 | 1.200 | 1.200 | 1.200 |
| **Vitamin C** | 0.02 | 0.020 | 0.020 | 0.020 | 0.020 | 0.020 |
| **Sum** | 100.00 | 84.945 | 100.000 | 100.000 | 100.000 | 100.000 |

### Experimental Example 1: Measurement of solubility

The solubility was measured when a beverage was prepared using the powders for preparing milk tea of Comparative Example and Examples 1 to 5 prepared in the Example above.

Specifically, 100 mL of purified water at 10°C, 35°C, and 55°C were prepared and poured into the respective beakers, and the beakers were placed in a stirrer (Thermo Scientific, Poly15), then 10 g of each sample of Comparative Example and Examples 1 to 5 prepared in the Example above was added and stirred at 200 rpm, and the time taken until the sample was completely dissolved was measured.

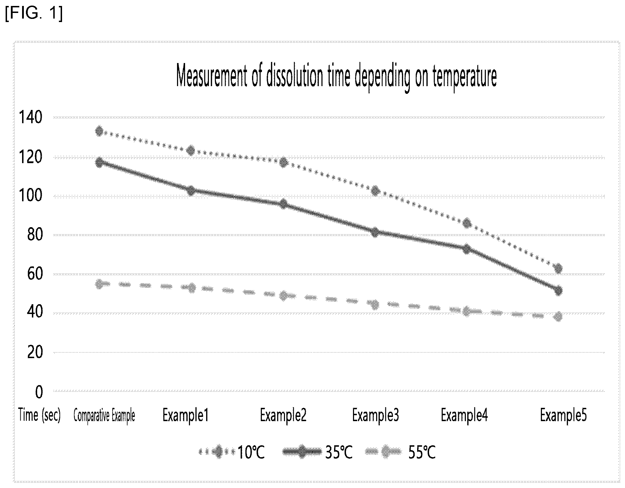

As a result, as illustrated in FIG. 1, it was found that the samples of Comparative Example and Examples 1 to 5 added to purified water at 55°C were all dissolved at a relatively fast rate, and it was found that the dissolution rate and dispersion rate were faster particularly as the sugar content was lower and the amount of sugar alcohol added was larger. In addition, it was found that a similar tendency was observed under low temperature conditions of 10°C and 35°C as well.

Through this, it has been found that the complete dispersion and dissolution rates of reduced sugar instant milk tea powders increase and the beverage preparation rate significantly increases although there may be some differences depending on the specifications and conditions of the mixer when an instant milk tea powder beverage is prepared. This suggests that efficiency can be improved in cafes and restaurants in which beverages are prepared and sold, and consumer convenience can be improved.

### Experimental Example 2: Sensory evaluation

The solubility was measured when a beverage was prepared using the powders for preparing milk tea of the Comparative Example and Examples 1 to 5 prepared in the Example above.

Specifically, milk tea prepared by mixing the powders for preparing milk tea of the Comparative Example and Examples 1 to 5 prepared in the Example above with milk was evaluated for each item by 40 trained male and female panelists to compare the sensory quality.

More specifically, for sensory evaluation, 20 g of the instant milk tea powder prepared above was weighed and mixed with 200 mL of warm milk to prepare milk tea, the prepared beverage was provided to the panelists, and the panelists were asked to compare the sensory quality. As the evaluation method, each sample, numbered with a three-digit random number table, was prepared, and one of the samples was randomly selected and provided to each panelist to consume, and then the panelist was asked to express the given attribute using a 5-point scale. This was repeated to evaluate all prepared samples, and the evaluated scores were analyzed in conformity to the T-test between the samples of Comparative Example and Experimental Examples, and then a statistically significant difference was examined (p<0.05).

**[Table 2]**

| | **Sweet taste** | **Bitter taste** | **Astringent taste** | **Body** | **Off-flavor** |
|---|---|---|---|---|---|
| **Comparative Example** | 4.18 | 4.3 | 3.6 | 3.6 | 2.1 |
| **Example 1** | 4.08 | 4.5 | 3.7 | 3.4 | 2.3 |
| **Example 2** | 4.21 | 4.38 | 3.5 | 3.85 | 1.89 |
| **Example 3** | 4.15 | 4.21 | 3.25 | 3.7 | 1.86 |
| **Example 4** | 4.3 | 3.7 | 2.8 | 3.9 | 1.85 |
| **Example 5** | 4.2 | 3.5 | 2.5 | 4.2 | 1.65 |

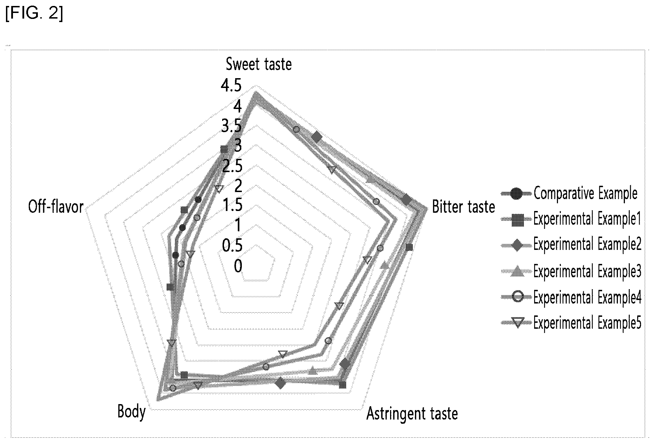

As a result, as shown in Table 2 and FIG. 2, it was found that the bitter and astringent tastes peculiar to black tea were significantly high in Comparative Example in which sugar was used. It was found that in Examples 4 and 5 of the present disclosure, in which a part of sugar was replaced with sugar alcohol, enzyme-treated stevia, and stevioside, the bitter and astringent tastes were reduced by 14% to about 20% and about 23% to about 31%, respectively, compared to those in Comparative Example.

Through this, it has been found that the bitter and astringent negative taste of existing instant milk tea powder is masked by reducing sugar content.

Based on the above description, it will be understood by those skilled in the art that the present disclosure may be implemented in a different specific form without changing the technical spirit or essential characteristics thereof. In this regard, it should be understood that the above embodiment is not limitative, but illustrative in all aspects. The scope of the disclosure is defined by the appended claims rather than by the description preceding them, and therefore all changes and modifications that fall within the metes and bounds of the claims, or equivalents of such metes and bounds are therefore intended to be embraced by the claims.

## Claims

1. A composition for preparing milk tea comprising black tea extract powder, xylose sugar, sugar alcohol, and a natural high-intensity sweetener, wherein
the xylose sugar and the natural high-intensity sweetener are comprised in a form of a core-shell structure in which the xylose sugar is coated with the natural high-intensity sweetener.

2. The composition according to claim 1, wherein
the black tea extract powder is comprised at 1 to 10 parts by weight based on 100 parts by weight of a total weight of the composition,
the xylose sugar is comprised at 30 to 90 parts by weight based on 100 parts by weight of a total weight of the composition,
the sugar alcohol is comprised at 0.1 to 50 parts by weight based on 100 parts by weight of a total weight of the composition, and
the natural high-intensity sweetener is comprised at 0.01 to 0.1 parts by weight based on 100 parts by weight of a total weight of the composition.

3. The composition according to claim 1, wherein the sugar alcohol is one or more selected from the group consisting of erythritol, tagatose, xylose, arabinose, ribose, xylitol, lactitol, maltitol, and sorbitol.

4. The composition according to claim 1, wherein the natural high-intensity sweetener is one or more selected from the group consisting of enzyme-treated stevia, stevioside, sucralose, aspartame, a monk fruit extract, a licorice extract, and thaumatin.

5. The composition according to claim 1, wherein the composition further comprises maltodextrin, a flavoring agent, or vitamin C.

6. The composition according to claim 1, wherein the composition is in a powder form.

7. Milk tea comprising the composition according to any one of claims 1 to 6.

8. The milk tea according to claim 7, wherein the milk tea has reduced bitter and astringent tastes compared to milk tea that does not comprise xylose sugar, sugar alcohol, and a natural high-intensity sweetener.

9. The milk tea according to claim 7, wherein the milk tea has improved solubility and dispersibility and mouthfeel compared to milk tea that does not comprise the core-shell structure.

10. A method for preparing a composition for preparing milk tea, the method comprising the steps of:
preparing a diluted solution by dissolving maltodextrin and a natural high-intensity sweetener in water;
preparing a core-shell structure in which xylose sugar is present as a core and the core is coated with the natural high-intensity sweetener by spraying and coating the xylose sugar with the diluted solution;
drying the core-shell with hot air in a temperature range of 70°C to 80°C for 10 to 30 minutes;
cooling the core-shell through treatment with cooling air at 1°C to 50°C for 10 to 30 minutes; and
preparing a composition by stirring the cooled core-shell, sugar alcohol, and black tea extract powder.

11. The method according to claim 10, wherein
the black tea extract powder is comprised at 1 to 10 parts by weight based on 100 parts by weight of a total weight of the composition,
the xylose sugar is comprised at 30 to 90 parts by weight based on 100 parts by weight of a total weight of the composition,
the sugar alcohol is comprised at 0.1 to 50 parts by weight based on 100 parts by weight of a total weight of the composition, and
the natural high-intensity sweetener is comprised at 0.01 to 0.1 parts by weight based on 100 parts by weight of a total weight of the composition.

12. The method according to claim 10, wherein the step of preparing a composition by stirring the cooled core-shell, sugar alcohol, and black tea extract powder further comprises adding maltodextrin, a flavoring agent, or vitamin C.

13. A composition for preparing milk tea prepared by the method according to claim 10.

14. Milk tea comprising the composition according to claim 13.

15. The milk tea according to claim 14, wherein the milk tea has reduced bitter and astringent tastes compared to milk tea that does not comprise xylose sugar, sugar alcohol, and a natural high-intensity sweetener.

16. The milk tea according to claim 14, wherein the milk tea has improved solubility and dispersibility and mouthfeel compared to milk tea comprising a composition prepared by a method that does not comprise a spraying and coating process.
